# EUROPÄISCHE PATENTSCHRIFT

(11) **EP 0 751 136 B1**
(45) Veröffentlichungstag und Bekanntmachung des Hinweises auf die Patenterteilung: **29.12.1999**
(21) Anmeldenummer: 96810417.4
(22) Anmeldetag: 20.06.1996
(51) Int. Cl.: C07D 305/08

(54) **Verfahren zur Herstellung von 3-hydroxyoxetanen**
Process for the preparation of 3-hydroxyoxetanes
Procédé pour la préparation de 3-hydroxyoxétanes

(30) Priorität: 29.06.1995 CH 191395
(43) Veröffentlichungstag der Anmeldung: 02.01.1997
(73) Patentinhaber: Novartis AG, 4058 Basel (CH); Novartis-Erfindungen Verwaltungsgesellschaft m.b.H., 1235 Wien (AT)
(72) Erfinder: Stutz, Wolfgang, Dr., 4333 Münchwilen (CH); Waditschatka, Rudolf, Dr., 5073 Gipf-Oberfrick (CH); Winter, Klas, 1891 Massongex (CH); von Frieling, Matthias, Dr., 79114 Freiburg (DE); Gressly, Rémy, 1897 Le Bouveret (CH); Jau, Beat, 4147 Aesch (CH); Bürki, Sébastian, 1870 Monthey (CH)

(56) Entgegenhaltungen:
- US-A- 4 395 561
- US-A- 5 209 771
- US-A- 5 342 823

## Beschreibung

Die vorliegende Erfindung betrifft ein neues Verfahren zur Herstellung von 3-Hydroxyoxetanen.

In US-A-4,395,561 ist ein Verfahren zur Herstellung von 3-Hydroxyoxetan beschrieben. Gemäss diesem Verfahren geht man von Carbonsäuren der Formel CH₃(CH₂)ₙCO₂H aus, worin n 0, 1, 2 oder 3 ist, und setzt diese in Gegenwart von FeCl₃ mit Epichlorhydrin zu einem Ester der Formel CH₃(CH₂)ₙCO-O-CH₂CH(OH)CH₂-Cl um. Dieser Ester wird durch basische Hydrolyse zu einem Oxetan cyclisiert, nachdem die Hydroxygruppe des Esters mit einer gegen die Einwirkung von Basen beständigen Gruppe geschützt wurde. Diese Schutzgruppe wird nach der Hydrolyse bzw. Cyclisierung durch Reaktion mit einer Säure wieder entfernt. Das so erhaltene 3-Hydroxyoxetan isoliert man dann durch Extraktion und/oder Destillation.

Ueberraschenderweise wurde nun gefunden, dass man dieses Verfahren deutlich verbessern kann, wenn an Stelle der genannten unverzweigten Carbonsäuren der Formel CH₃(CH₂)ₙCO₂H solche der Formel R-CO₂H eingesetzt werden, worin R verzweigtes Alkyl ist.

Gegenstand der vorliegenden Erfindung ist somit ein Verfahren zur Herstellung von 3-Hydroxyoxetanen der Formel I 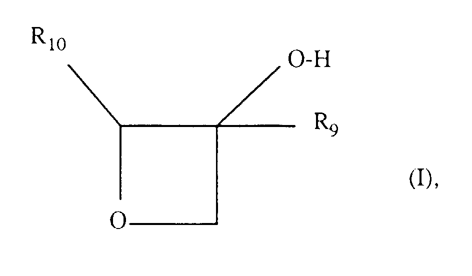 worin R₉ und R₁₀ unabhängig voneinander Wasserstoff oder C₁-C₄-Alkyl sind, durch
(1) Umsetzung einer Carbonsäure R-CO₂H, worin R verzweigtes Alkyl ist, mit einem Epichlorhydrin der Formel II 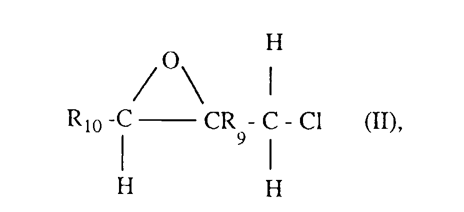 worin R₉ und R₁₀ die angegebenen Bedeutungen haben, zu einem Ester der Formel III 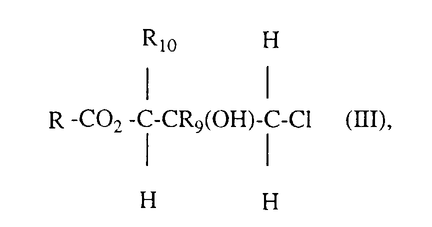 worin R, R₉ und R₁₀ die angegebenen Bedeutungen haben,
(2) Umsetzung dieses Esters mit einem Aether der Formel IV

   CHR₁=CH-O-R₂ (IV),

   worin R₁ Wasserstoff oder Methyl, R₂ C₁-C₆-Alkyl ist, oder R₁ und R₂ zusammen einen Rest der Formel -(CH₂)₃- bilden, in Gegenwart eines Katalysators zu einem Ester der Formel V 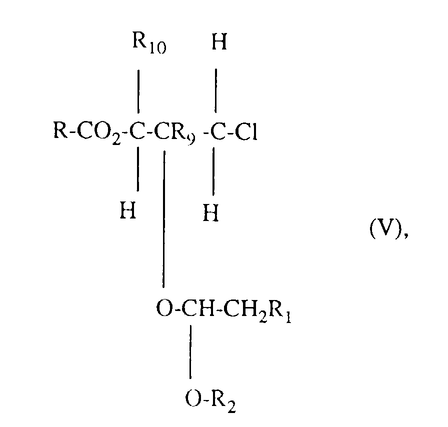
(3) Hydrolyse und Cyclisierung dieses Esters in Gegenwart einer Base zur Verbindung der Formel VI 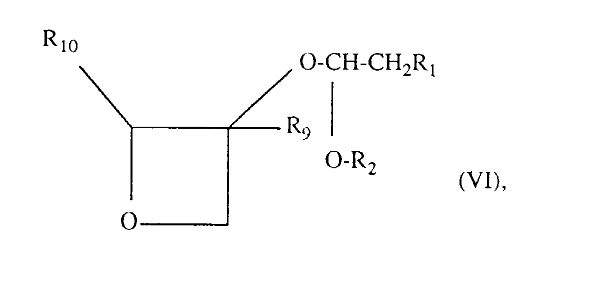 worin R₁, R₂, R₉ und R₁₀ die angegebenen Bedeutungen haben,
(4) Acetalspaltung in Gegenwart einer Säure zum entsprechenden 3-Hydroxyoxetan und
(5) Isolierung dieses 3-Hydroxyoxetans.

In Stufe (1) des erfindungsgemässen Verfahrens werden bevorzugt solche Carbonsäuren R-CO₂H verwendet, worin der Alkylrest R eine Verzweigung am α-Kohlenstoffatom aufweist. Insbesondere ist R ein α-verzweigter Alkylrest mit 3 bis 8 Kohlenstoffatomen, wovon beispielsweise 1-Aethylpentyl und t-Butyl und vor allem 1-Aethylpropyl besonders geeignet sind. Säuren mit cyclischen Alkylresten wie Cyclopentyl, Cyclohexyl und Cyclooctyl können ebenfalls mit Vorteil im erfindungsgemässen Verfahren eingesetzt werden.

Bevorzugt erfolgt die Addition der Carbonsäure an ein Epichlorhydrin in Gegenwart eines Katalysators. Dieser kann sauer oder basisch sein. Als saure Katalysatoren kommen beispielsweise Lewis-Säuren in Frage wie das in US-A-4,395,561 genannte FeCl₃, saure Gesteinsmehle wie Montmorillonite und auch die üblichen Mineralsäuren wie Chlor- und Bromwasserstoff, Phosphor-, Salpeter- und Schwefelsäure. Ferner eignen sich für die erste Reaktionsstufe auch Phasentransferkatalysatoren. Als Beispiele hierfür seien Tetrabutylammoniumchlorid, 3-(1-Pyridino)-1-propansulfonat und Pyridintosylat genannt. Das erfindungsgemässe Verfahren wird aber mit Vorteil in Gegenwart eines basischen Katalysators ausgeführt. Beispielsweise können Alkalimetallhydroxide wie Natrium- und Kaliumhydroxid verwendet werden. Besonders eignen sich aber Trialkylamine wie Triäthyl- und Tributylamin, N,N-Dialkylaniline wie N,N-Diäthylanilin, und substituierte Pyridine, vor allem mit Alkyl- oder Dialkylaminogruppen substituierte Pyridine, beispielsweise 2-Methyl- und 2,6-Dimethylpyridin sowie N,N-Dimethylaminopyridin. Die günstigsten Resultate werden mit Pyridin selbst erreicht. Ferner können auch polycyclische, insbesondere bicyclische Amidine eingesetzt werden wie DBU und DBN, die beispielsweise in Synthesis, 591, 1972, beschrieben sind, und insbesondere auch auf Polymere gebundene Pyridine und alkylierte Aminopyridine wie Poly-DMAP.

Zur Einführung der Schutzgruppe in Stufe (2) haben sich besonders die Vinylätherderivate der Formel IV bewährt. Der Substituent R₂ in Formel IV ist z.B. Methyl, Aethyl, Propyl, Butyl, Pentyl oder Hexyl oder ein verzweigtes Isomere davon. Ferner kann R₂ zusammen mit R₁ einen -(CH₂)₃- Rest bilden und so einen Dihydropyranring bilden. Zur Verwendung im erfindungsgemässen Verfahren besonders geeignete Vinyläther sind solche, worin R₁ Wasserstoff und R₂ C₂-C₅-Alkyl ist, wie z.B. Aethyl-, Butyl-, Pentyl- und insbesondere Isobutylvinyläther, ferner auch Methylisopropenyläther.

Die Umsetzung des Esters der Formel III mit dem Vinyläther der Formel IV in Stufe (2) erfolgt in Gegenwart eines Katalysators. Hier kommen insbesondere Alkyl- und Arylsulfonsäuren sowie ihre Salze in Frage. Als bevorzugte Beispiele seien Methansulfonsäure und p-Toluolsulfonsäure genannt. Gute Resultate werden aber auch mit halogenierten Carbonsäuren wie Trifluor- und Trichloressigsäure sowie den oben genannten Mineralsäuren erzielt, insbesondere dann, wenn diese wasserfrei sind. Ferner können auch saure Ionenaustauschharze wie Dowex® eingesetzt werden.

Die Hydrolyse und Cyclisierung des geschützten Esters der Formel V zum geschützten 3-Hydroxyoxetan in Stufe (3) verläuft in alkalischem Medium. Als Base kann grundsätzlich jede Verbindung gewählt werden, die in Lösungsmitteln, insbesondere polaren, Hydroxylionen freizusetzen vermag, insbesondere Natrium- und Kaliumhydroxid sowie entsprechende Methanolate. Bevorzugte polare Lösungsmittel sind z.B. Alkohole, DMF, DMA, N-Methylpyrrolidon, Dioxan und insbesondere Wasser.

Die Abspaltung der Schutzgruppe in Stufe (4), eine Acetalspaltung, wird durch Umsetzung des geschützten 3-Hydroxyoxetans mit einer Säure in einem protischen Lösungsmittel wie Alkoholen und insbesondere Wasser erreicht. Beispielsweise eignen sich die oben für die Verwendung in Stufe (2) erwähnten Säuren, wobei Mineralsäuren wie Chlor-, Bromwasserstoff, Schwefel-, Salpeter- und Phosphorsäure eine besondere Bedeutung zukommt. Schwefelsäure und Chlorwasserstoff sind ganz besonders bevorzugt. Nach der Acetalspaltung kann die Reaktionsmischung, insbesondere bei der satzweisen Reaktionsführung, mit Basen wie z.B. Alkalimetall- oder Erdalkalimetallhydroxiden neutralisiert werden.

Das erfindungsgemässe Verfahren lässt sich sowohl satzweise (diskontinuierlich, batchweise) als auch kontinuierlich durchführen.

Das satzweise Verfahren erfolgt bevorzugt in üblichen Rührkesseln sowie, im Hinblick auf die letzte Stufe, in üblichen Füllkörperkolonnen. In einer Variante des erfindungsgemässen satzweisen Verfahrens können die Reaktionsstufen (1), (2) und (3) als Eintopfreaktion zusammengefasst und das Reaktionsprodukt der (3). Stufe, das geschützte 3-Hydroxyoxetan der Formel (VI), durch azeotropes Abdestillieren oder Wasserdampfdestillation angereichert werden.

Die kontinuierliche Verfahrensführung ist bevorzugt. Im kontinuierlichen Verfahren verwendet man für die Durchführung der Stufe (1) vorzugsweise einen Schlaufenreaktor. Dadurch wird eine gleichmässige Verteilung der Reaktionswärme über den gesamten Reaktor gewährleistet, und die turbulente Strömung ermöglicht die Einstellung eines hohen Rücklaufverhältnisses. Zur Erreichung eines hohen Umsatzes kann dem Schlaufenreaktor ein Rohrreaktor in Reihe nachgeschaltet werden. Der Schlaufenreaktor wird vorzugsweise bei Temperaturen zwischen 90 und 130°C betrieben. Die mittlere Verweilzeit liegt dann in einem Bereich von etwa 40 bis 60 Minuten. Die Betriebstemperatur des nachgeschalteten Rohrreaktors liegt in der Regel etwas höher, beispielsweise zwischen 110 und 130°C, und die mittlere Verweilzeit beträgt darin etwa 10 bis 20 Minuten. Ueblicherweise werden so in Stufe (1) Ausbeuten um 80% an Verbindung der Formel III erzielt. Die Einführung der Schutzgruppe in Stufe (2) wird vorzugsweise in einem Rührkessel vorgenommen, da eine besonders gute Durchmischung der Reaktionsmasse erforderlich ist. Bei Reaktionstemperaturen von 40 bis 55°C und mittleren Verweilzeiten von 15 bis 25 Minuten können die Verbindungen der Formel V in Ausbeuten um 95% erhalten werden. Für die Ausführung der Stufe (3) hat sich ein Mehrkammerreaktor als vorteilhaft erwiesen. Man wählt vorzugsweise Temperaturen von 140 bis 150°C, die bei einem Druck von etwa 3 bis 4 bar erreicht werden können. Um bei diesen Temperaturen über dem Sättigungsdruck der Reaktionsmasse arbeiten zu können, wird vorzugsweise ein Stickstoffpolster überlagert, mit dem ein Gesamtdruck von etwa 5 bar im Reaktor eingestellt wird. Die mittlere Verweilzeit der Reaktionsmasse im Reaktor beträgt üblicherweise 50 bis 70 Minuten. Durch Wasserdampfdestillation lässt sich die Verbindung der Formel VI als bis etwa 80%ige wässrige Lösung anreichern. Zur Wasserdampfdestillation verwendet man mit Vorteil eine Kolonne mit Schikanen, beispielsweise Konus/Trichter-Schikanen oder eine Rührkesselkaskade aus beispielsweise 3 Rührkesseln, durch die im Gegenstrom Wasserdampf unter Niveau durchgeleitet wird. Die Verbindungen der Formel VI lassen sich so in Ausbeuten von mehr als 85% erhalten. Die Ausbeuten über alle 3 Stufen liegen somit üblicherweise im Bereich von 64 bis 70%. Die Spaltung der Verbindungen der Formel VI zu den entsprechenden 3-Hydroxyoxetanen in Stufe (4) kann beispielsweise in einem Rührkessel oder in einer Rührkesselkaskade mit etwa 2 Rührkesseln durchgeführt werden. Die Reaktionstemperaturen sollten dann in einem Bereich von 50 bis 70°C liegen und die mittleren Verweilzeiten bei etwa 1,5 bis 3 Stunden pro Kessel (3 bis 6 Stunden Totalverweilzeit). Vorzugsweise arbeitet man mit Unterdruck, beispielweise 0,2 bis 0,5 bar, um den bei der Spaltung gebildeten Acetaldehyd und zumindest teilweise auch Isobutanol und andere in geringer Konzentration vorliegende leichtsiedende Nebenprodukte aus dem Reaktionsgemisch zu entfernen. Die 3-Hydroxyoxetane fallen als im wesentlichen wässrige Lösung an mit einem Gehalt von etwa 15 bis 25%. Die Ausbeuten liegen im allgemeinen bei 90 bis 95%. Die Reindarstellung der 3-Hydroxyoxetane erfolgt in Stufe (5) vorzugsweise durch Rektifizierung der genannten wässrigen Lösung des Rohprodukts aus Stufe (4). Es ist vorteilhaft, bei Unterdruck, z. B. 100 mbar, in einer ersten Kolonne die leichtflüchtigen Anteile wie Wasser und Alkohole (entsprechend den Bedeutungen von R₁ und R₂), insbesondere Isobutanol über Kopf abzudestillieren. Das Sumpfprodukt kann dann in eine zweite Kolonne eingespeist werden, wo die Auftrennung in hochsiedende Nebenprodukte und 3-Hydroxyoxetan (Kopfprodukt) erfolgt. 3-Hydroxyoxetane lassen sich auf diese Weise in Reinheiten von über 95% und Ausbeuten von 95% und mehr gewinnen.

Die Ausbeute an 3-Hydroxyoxetanen über alle Stufen beträgt 60 bis 66%.

In einer Variante des erfindungsgemässen kontinuierlichen Verfahrens kann der Rektifizierung eine Extraktion vorangehen, um eine Produktlösung mit deutlich vermindertem Salzgehalt zu erhalten. Vorzugsweise wird als Extraktionsmittel das in der Produktlösung ohnehin schon vorhandene Lösungsmittel (entsprechend den Resten R₁ und R₂), insbesondere Isobutanol verwendet. Es wird kontinuierlich bei der Phasentrennung des Kopfproduktes in der ersten Kolonne gewonnen. Die Extraktion kann in allen üblichen Extraktionsapparaten erfolgen, vorzugsweise in einer mehrstufigen gerührten Extraktionskolonne.

Das erfindungsgemässe Verfahren, mit dem bevorzugt unsubstituierte 3-Hydroxyoxetane hergestellt werden, weist gegenüber dem in US-A-4,395,561 offenbarten Verfahren die folgenden Vorteile auf:
- Ausbeute, die sich gegenüber dem Verfahren nach US-A-4,395,561 (47%) um mindestens 40% auf 65% und mehr steigern lässt; die in Stufe (3) besonders zu erwähnende Ausbeutesteigerung ist hauptsächlich auf die Verwendung verzweigter Carbonsäuren zurückzuführen;
- das am Ende der Stufe (1) vorliegende Isomerenverhältnis von Verbindung der Formel III zu Verbindung der Formel IIIa 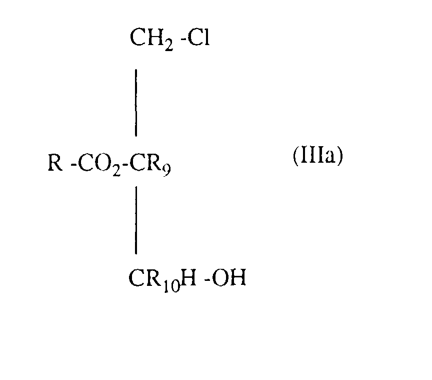 liegt deutlich auf der Seite der Verbindung der Formel III, was die Ausbeute und die Reinheit des Produktes erhöht;
- Reinheit des Produktes, die 95% und mehr betragen kann;
- Effizienz des Verfahrens, die durch eine wesentliche Verkürzung der Reaktionszeiten gesteigert werden kann;
- Verfahrenssicherheit, insbesondere im kontinuierlichen Betrieb, wo mit 20 mal kleineren Reaktionsgefässen, d.h. kleineren Mengen Reaktionsmischung gearbeitet werden kann, um dieselbe Produktmenge wie im Satzbetrieb zu erhalten; die Tragweite im Ereignisfall wird somit deutlich reduziert. Es ist auch zu berücksichtigen, dass vor allem die destillative Reinigung in der 5. Stufe bei Temperaturen von 130 bis 150°C durchgeführt wird, wo eine thermische Zersetzung des 3-Hydroxyoxetans bereits in 4 Stunden erfolgen kann. Hier ist eine satzweise Verfahrensführung infolge hoher Verweilzeiten nachteilig;
- reproduzierbare Qualität des Produkts, insbesondere im kontinuierlichen Betrieb, wo einmal eingestellte Verfahrensparameter solange konstant bleiben, bis die Anlage abgestellt wird. Im Satzbetrieb ist es möglich, dass selbst geringfügig veränderte Parameter zu unterschiedlichen Produktqualitäten führen können;
- die verzweigten Carbonsäuren sind wesentlich besser recyclierbar als die nicht verzweigten.

Die erfindungsgemäss hergestellten 3-Hydroxyoxetane werden speziell als Ausgangsprodukte bei der Herstellung von Sulfonylharnstoffherbiziden verwendet, wie sie beispielsweise in US-A-5,209,771 und US-A-5,342,823 beschrieben sind. In einem ersten Schritt werden erfindungsgemäss hergestellte 3-Hydroxyoxetane z.B. mit einem Säurechlorid der Formel VIII

Q― COCl (VIII),

worin Q ein Rest der Formel 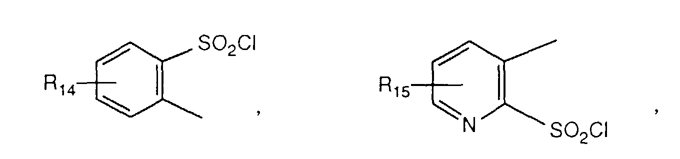 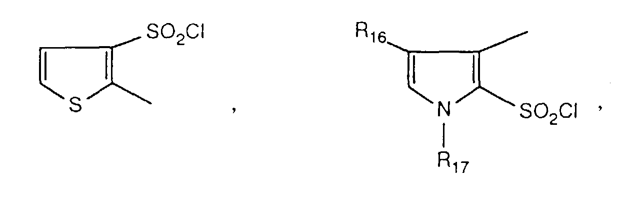 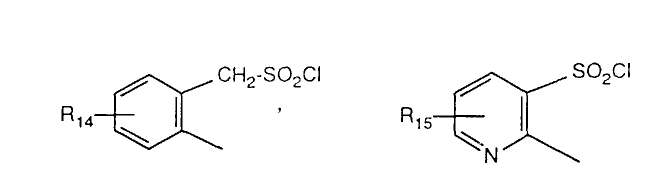 oder 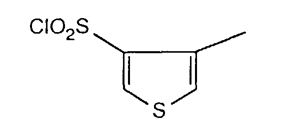 ist, worin R₁₄ Wasserstoff, Fluor, Chlor, Brom, Jod, - (X)ₙR₃, worin X Sauerstoff, Schwefel, SO oder SO₂, R₃ C₁-C₄-Alkyl, durch 1 bis 4 Halogenatome, C₁-C₃-Alkoxy oder C₁-C₃Alkylthio substituiertes C₁-C₄-Alkyl ist, oder R₃ C₂-C₄-Alkenyl oder durch 1 bis 4 Halogenatome substituiertes C₂-C₄ Alkenyl und n 0 oder 1 ist, oder R₁₄ Nitro, NR₄R₅, worin R₄ Wasserstoff, Methoxy, Aethoxy oder C₁-C₃-Alkyl und R₅ Wasserstoff oder C₁-C₃-Alkyl ist, oder R₁₄ -CCR₆, worin R₆ Wasserstoff, Methyl oder Aethyl ist, oder R₁₄ -O-CHR₇-CCR₆, worin R₆ Wasserstoff, Methyl oder Aethyl und R₇ Wasserstoff oder Methyl ist, oder R₁₄ Cyano ist, R₁₅ Wasserstoff, Fluor, Chlor, C₁-C₄-Alkyl oder Methoxy ist, R₁₆ Wasserstoff, Fluor oder Chlor ist und R₁₇ Methyl oder 2-Pyridyl ist, zu Verbindungen der Formel VII 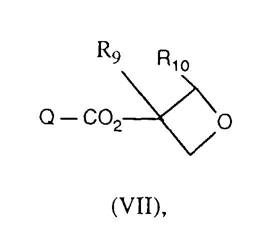 umgesetzt, worin R₉ und R₁₀ die angegebene Bedeutung haben, welche dann als Zwischenprodukt durch übliche und in US-A-5,209,771 und US-A-5,342,823 beschriebene Methoden wie Ueberführung in das entsprechende Sulfonamid und weitere Umsetzung mit einem Pyridyl-, Pyrimidyl-, Triazolyl- oder Triazinylphenylcarbamat oder einem Pyridyl-, Pyrimidyl-, Triazolyl- oder Triazinylisocyanat in die entsprechenden Sulfonylharnstoffherbizide überführbar sind.

Um die Reinheit der so herstellbaren Sulfonylharnstoffherbizide zu erhöhen, können die erfindungsgemäss hergestellten 3-Hydroxyoxetane unmittelbar vor ihrer Umsetzung mit dem Säurechlorid der Formel VIII mit katalytischen Mengen eines vorzugsweise zweiwertigen Metallsalzes, insbesondere eines Chlorids wie z.B. Magnesiumchlorid oder Calciumchlorid, behandelt werden.

Mit den erfindungsgemäss hergestellten 3-Hydroxyoxetanen werden bevorzugt die Zwischenprodukte der Formel VIIa 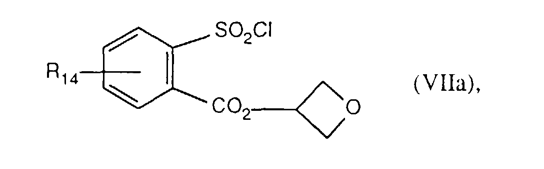 worin R₁₄ die oben angegebene Bedeutung hat, und insbesondere der Formel VIIb 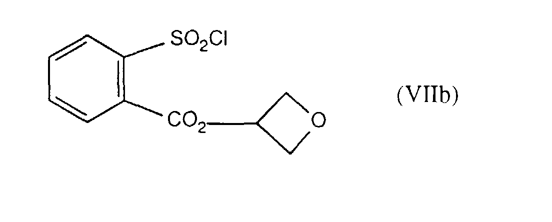 zugänglich.

Die nachfolgenden Beispiele erläutern die Erfindung weiter.

### Beispiel 1: Satzweise Herstellung von 3-Hydroxyoxetan

Stufe (1): Eine Mischung von 2,53g Pyridin und 383g 2-Aethylbuttersäure wird in einem 750ml Sulfierkolben bei 96 bis 100°C während 4 Stunden tropfenweise mit 296g Epichlorhydrin versetzt. Anschliessend lässt man 1 Stunde bei dieser Temperatur weiterreagieren, senkt innerhalb von 3 Stunden die Temperatur auf 73 bis 76°C und lässt die Reaktionsmischung weitere 2 Stunden bei dieser Temperatur. Das gaschromatographisch ermittelte Verhältnis von Verbindung der Formel III zu Verbindung der Formel IIIa, worin R₉ und R₁₀ Wasserstoff bedeuten und R 1-Aethylpropyl ist, beträgt 91:9.
Stufe (2): Die Reaktionsmischung aus Stufe (1) und 5,23g Methansulfonsäure werden bei 68 bis 71°C während 5 Stunden tropfenweise mit 353g Isobutylvinyläther in einem 1,51 Sulfierkolben versetzt. Anschliessend lässt man bei dieser Temperatur 3 Stunden lang ausreagieren.
Stufe (3): Die Reaktionsmischung aus Stufe (2) wird während 4 Stunden bei 110 bis 120°C zu einer Mischung aus 736g einer wässrigen 40%igen Natriumhydroxidlösung und 10g 2-Aethylbuttersäure in einem 2,51 Sulfierkolben getropft. Durch azeotropes Abdestillieren, kontinuierliches Ersetzen des entfernten Wassers im Reaktionsgemisch und Abtrennen der wässrigen Phase im Destillat erhält man 475g organische Phase mit einem Gehalt von 77,9% Isobutoxy-äthoxy-oxetan.
Stufe (4): Eine Mischung von 446g von in Stufe (3) erhaltenem Isobutoxy-äthoxy-oxetan und 400g Wasser werden in einem 1,51 Sulfierkolben bei 20 bis 25°C tropfenweise mit 10%iger Schwefelsäure versetzt bis sich ein pH-Wert von 2,5 bis 3 einstellt. Dann wird auf 300mbar evakuiert und auf 65 bis 70°C erhitzt, um Acetaldehyd aus der Reaktionsmischung zu entfernen. Nun kann entweder durch Zudosieren von 25%iger Kaliumhydroxidlösung der pH-Wert auf 8,5 bis 9,0 eingestellt, die Reaktionsmischung 1 Stunde bei 65 bis 70°C belassen, anschliessend auf 20 bis 25°C abgekühlt und mit Schwefelsäure auf einen pH-Wert von 7 bis 7,5 eingestellt werden, oder man lässt auf 20 bis 25°C abkühlen und stellt dann durch Zugabe von Magnesium- oder Calciumhydroxidlösung einen pH-Wert von 7 ein.
Stufe (5): Aus der Reaktionsmischung aus Stufe (4) werden durch fraktionierte Destillation über eine Füllkörperkolonne Wasser und leichtsiedende organische Nebenprodukte entfernt. Der verbleibende Rückstand wird über eine Kurzwegdestillationsapparatur destilliert. Man erhält so 146g 3-Hydroxyoxetan (96%ig).

Die Ausbeute an 3-Hydroxyoxetan über alle Stufen beträgt 63%.

### Beispiel 2: Satzweise Herstellung von 3-Hydroxyoxetan

Stufe (1): Eine Mischung von 8,1g wasserfreiem Eisen-III-chlorid und 592,8g 2-Aethylbuttersäure wird in einem 2,51 Sulfierkolben bei 80 bis 85°C während 3,5 Stunden tropfenweise mit 485,6g Epichlorhydrin versetzt. Anschliessend lässt man 2 Stunden bei dieser Temperatur weiterreagieren. Das gaschromatographisch ermittelte Verhältnis von Verbindung der Formel III zu Verbindung der Formel IIIa, worin R₉ und R₁₀ Wasserstoff bedeuten und R 1-Aethylpropyl ist, beträgt 93:7.
Stufe (2): Die Reaktionsmischung aus Stufe (1) und 3,7g Methansulfonsäure werden bei 68 bis 71°C während 5 Stunden tropfenweise mit 551g Isobutylvinyläther in einem 2,51 Sulfierkolben versetzt. Anschliessend lässt man bei dieser Temperatur 3 Stunden lang ausreagieren. Der Umsatz in dieser Stufe beträgt über 96%.
Stufe (3): Die Reaktionsmischung aus Stufe (2) wird während 4 Stunden bei 110 bis 120°C zu einer Mischung aus 1150g einer wässrigen 40%igen Natriumhydroxidlösung und 15g 2-Aethylbuttersäure in einem 4,5l Sulfierkolben getropft. Durch azeotropes Abdestillieren, kontinuierliches Ersetzen des entfernten Wassers im Reaktionsgemisch und Abtrennen der wässrigen Phase im Destillat erhält man 816g organische Phase mit einem Gehalt von 82% Isobutoxy-äthoxy-oxetan.
Stufe (4): Eine Mischung von 776g von in Stufe (3) erhaltenem Isobutoxy-äthoxy-oxetan und 700g Wasser wird wie in Beispiel 1, Stufe (4) beschrieben umgesetzt.
Stufe (5): Aus der Reaktionsmischung aus Stufe (4) werden durch fraktionierte Destillation über eine Füllkörperkolonne Wasser und leichtsiedende organische Nebenprodukte entfernt. Der verbleibende Rückstand wird über eine Kurzwegdestillationsapparatur destilliert. Man erhält so 258g 3-Hydroxyoxetan (96,2%ig).

Die Ausbeute an 3-Hydroxyoxetan über alle Stufen beträgt 70,5%.

### Beispiel 3: Satzweise Herstellung von 3-Hydroxy-3-methyl-oxetan

Stufe (1): Eine Mischung von 0,42g wasserfreiem Eisen-III-chlorid und 29,3g 2-Aethylbuttersäure wird in einem 100ml Sulfierkolben bei 80 bis 85°C während 4 Stunden tropfenweise mit 27,7g 2-Chlormethyl-2-methyl-oxiran versetzt. Anschliessend lässt man 27 Stunden bei dieser Temperatur weiterreagieren.
Stufe (2): Die Reaktionsmischung aus Stufe (1) und 0,147g Methansulfonsäure werden bei 68 bis 72°C während 4 Stunden tropfenweise mit 27,5g Isobutylvinyläther versetzt. Anschliessend lässt man bei dieser Temperatur 16 Stunden lang ausreagieren.
Stufe (3): Die Reaktionsmischung aus Stufe (2) wird während 3 Stunden bei 110 bis 120°C zu 57,5g einer wässrigen 40%igen Natriumhydroxidlösung getropft. Durch azeotropes Abdestillieren, kontinuierliches Ersetzen des entfernten Wassers im Reaktionsgemisch und Abtrennen der wässrigen Phase im Destillat erhält man 35,4g organische Phase mit einem Gehalt von 47,5% entsprechend 35,7% Ausbeute.
Stufe (4): Eine Mischung von 35,4g von in Stufe (3) erhaltenem Isobutoxy-äthoxy-methyl-oxetan und 27,5g Wasser werden bei 20 bis 25°C tropfenweise mit 10%iger Schwefelsäure versetzt bis sich ein pH-Wert von 2,5 bis 3 einstellt. Dann wird auf 300mbar evakuiert und auf 50 bis 70°C erwärmt, um Acetaldehyd aus der Reaktionsmischung zu entfernen. Durch Zudosieren von 25%iger Kaliumhydroxidlösung wird der pH-Wert auf 8,5 bis 9 eingestellt, 1 Stunde bei 65 bis 70°C belassen, anschliessend auf 20 bis 25°C abgekühlt und mit Schwefelsäure auf einen pH-Wert von 7 bis 7,5 eingestellt.
Stufe(5): Aus der Reaktionsmischung aus Stufe (4) werden durch fraktionierte Destillation über eine Füllkörperkolonne Wasser und leichtsiedende organische Nebenprodukte entfernt. Der verbleibende Rückstand von 9,5g enthält die Titelverbindung zu 61%.

Die Ausbeute an 3-Hydroxy-3-methyl-oxetan über alle Stufen beträgt 26,4%.

### Beispiel 4: Kontinuierliche Herstellung von 3-Hydroxyoxetan

Stufe (1):In einem 3,41 Schlaufenreaktor werden 2-Aethylbuttersäure und Epichlorhydrin im Ueberschuss (25mol%) sowie 1,5mol% Pyridin (bezogen auf 2-Aethylbuttersäure) bei einer Temperatur von 95 bis 105°C miteinander umgesetzt. Nach einer mittleren Verweilzeit von 45 Minuten wird die Reaktionsmischung in einen Rohrreaktor (Querschnitt: 38 mm, Nutzlänge: 1200 mm, Art der Füllkörper: Sulzer BX) geführt. Die Betriebstemperatur in diesem Reaktor beträgt 110 bis 120°C, die mittlere Verweilzeit 15 Minuten. Ueberschüssiges Epichlorhydrin wird bei 105°C und 200mbar entfernt. Man erhält den Chlorhydrinester in 80%iger Ausbeute.
Stufe (2): Die Reaktionsmischung aus Stufe (1) wird in einen kontinuierlich betriebenen 2,51 Rührkessel geleitet und dort bei einer Temperatur von 43°C mit Isobutylvinyläther (1,08 Aequivalente bezogen auf 2-Aethybuttersäure) umgesetzt. Gleichzeitig erfolgt die Zugabe von Methansulfonsäure in einer Menge von 0,024 Aequivalenten bezogen auf Isobutylvinyläther. Die mittlere Verweilzeit beträgt 20 Minuten. In dieser Stufe ist das Rühren sehr wichtig, da sich während der Reaktion laufend ein feiner weisser Niederschlag bildet, der zum grössten Teil aus Pyridiniumsalz besteht. Bezogen auf eingesetzten Ester erhält man das Produkt in 95%iger Ausbeute.
Stufe (3): Diese Stufe, Verseifung und Ringschluss, erfolgt in einem 10 l Mehrkammerreaktor bei einer Temperatur von 145°C und einem Druck von 3 bis 5 bar. 2,5 Aequivalente Natronlauge in Form einer auf 145°C erhitzten 35%igen wässrigen Lösung werden zudosiert. Die Verweilzeiten im Reaktor betragen 60 bis 80 Minuten. Das Produkt wird mittels Dampfdestillation vom Reaktionsgemisch abgetrennt. Dazu wird das Reaktionsgemisch durch eine Rührkesselkaskade von je 5 l Volumen geführt und 2 bis 2,5kg Wasserdampf/Kilo Reaktionsgemisch aus Stufe (3) im Gegenstrom unter Niveau eingeleitet Man erhält das Produkt dieser Stufe in 88%iger Ausbeute.
Stufe (4): In einer Reaktorkaskade von zwei 10 1-Reaktoren werden unter einem Druck von 0,3bar, bei einer Temperatur von 60°C und einer mittleren Verweilzeit von 4 bis 5 Stunden (total) kontinuierlich das Produkt aus Stufe (3) (2,18kg/Stunde) und Wasser (1,35kg/Stunde) zudosiert. Dabei wird der pH-Wert mit Chlorwasserstoffsäure auf 3,0 eingestellt. Die aus dem zweiten Rührkessel abfliessende Lösung wird mit Natronlauge auf einen pH-Wert von 7 bis 8 eingestellt. Die Ausbeute an Stufe (4)-Produkt beträgt 95%.
Stufe (5): Die am Ende der Stufe (4) anfallende Reaktionslösung wird durch Rektifizierung auf 2 Kolonnen aufgearbeitet. In der ersten Kolonne werden bei 40°C Kopftemperatur und 100 mbar Kopfdruck alle Stoffe mit einem niedrigeren Siedepunkt als 3-Hydroxyoxetan als Kopfprodukt abgetrennt, hauptsächlich Isobutanol und Wasser. Das 3-Hydroxyoxetan wird mit den höhersiedenden Komponenten bei 130°C in die zweite Kolonne eingespeist und bei 10mbar Kopfdruck und 73°C Kopftemperatur in mehr als 95%iger Reinheit als Kopfprodukt erhalten.

Die Gesamtausbeute an 3-Hydroxyoxetan über alle Stufen beträgt 60,5%.

### Beispiel 5: Herstellung der Verbindung der Formel VIIb

109,6g o-Sulfobenzoesäuremonoammoniumsalz, erhältlich durch saure Ringöffnung von Saccharin, werden in 500ml Toluol und 10ml DMF vorgelegt. Während 2 Stunden leitet man bei einer Temperatur von 70 bis 75°C 130g Phosgen ein. Danach rührt man noch 30 Minuten bei dieser Temperatur, erhöht dann die Temperatur innerhalb 1 Stunde auf 105 bis 110°C und hält die Reaktionsmischung noch etwa 1 Stunde bei dieser Temperatur bis die Gasentwicklung beendet ist. Unter Stickstoffstrom lässt man auf Raumtemperatur abkühlen. Man filtriert und wäscht den Rückstand mit Toluol. Das Filtrat wird vollständig eingedampft, und man erhält 120,4g der o-Sulfonsäure-benzoesäure-dichlorid in Form eines gelben Oels. 34g dieser Verbindung und 120,4g 3-Hydroxyoxetan, das gegebenenfalls mit einer katalytischen Menge von Magnesiumchlorid behandelt worden ist, werden in 350ml Methylenchlorid bei -5°C vorgelegt. Dann tropft man 34g Pyridin zu, wobei die Temperatur 0°C nicht übersteigt. Nach dem Zutropfen lässt man die Reaktionsmischung auf Raumtemperatur erwärmen und rührt noch 1 Stunde. Es hat sich eine Suspension gebildet, die mit Eiswasser versetzt wird. Die organische Phase wird abgetrennt, mit Eiswasser, kalter Natriumhydrogencarbonatlösung und nochmals mit Eiswasser gewaschen. Anschliessend trocknet man über Natriumsulfat und filtriert. Man erhält die Verbindung der Formel VIIb in 70%iger Ausbeute.

## Patentansprüche

1. Verfahren zur Herstellung von 3-Hydroxyoxetanen der Formel I 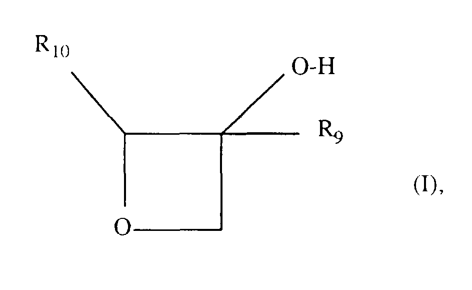 worin R₉ und R₁₀ unabhängig voneinander Wasserstoff oder C₁-C₄-Alkyl sind, durch
(1) Umsetzung einer Carbonsäure R-CO₂H, worin R verzweigtes Alkyl ist, mit einem Epichlorhydrin der Formel II 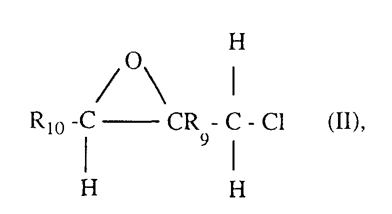 worin R₉ und R₁₀ die angegebenen Bedeutungen haben, zu einem Ester der Formel III 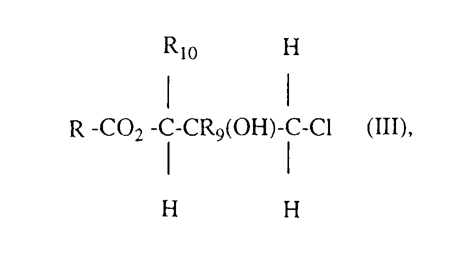 worin R, R₉ und R₁₀ die angegebenen Bedeutungen haben,
(2) Umsetzung dieses Esters mit einem Aether der Formel IV
CHR₁=CH-O-R₂ (IV),
worin R₁ Wasserstoff oder Methyl, R₂ C₁-C₆-Alkyl ist, oder R₁ und R₂ zusammen einen Rest der Formel -(CH₂)₃- bilden, in Gegenwart eines Katalysators zu einem Ester der Formel V 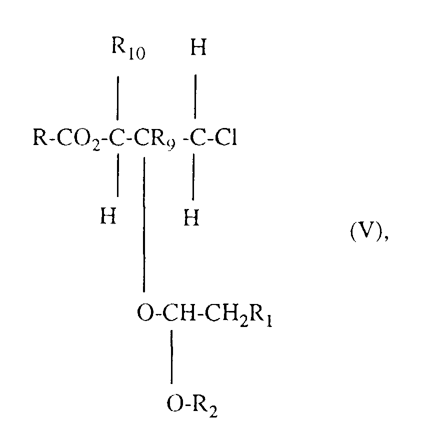
(3) Hydrolyse und Cyclisierung dieses Esters in Gegenwart einer Base zur Verbindung der Formel VI 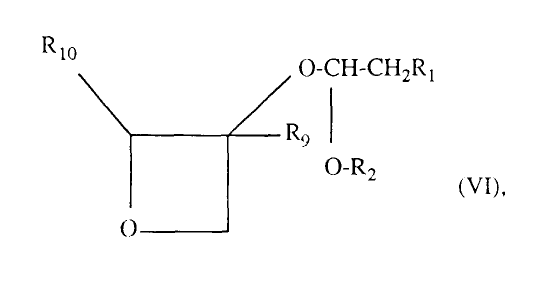 worin R₁, R₂, R₉ und R₁₀ die angegebenen Bedeutungen haben,
(4) Acetalspaltung in Gegenwart einer Säure zum entsprechenden 3-Hydroxyoxetan und
(5) Isolierung dieses 3-Hydroxyoxetans.

2. Verfahren nach Anspruch 1, dadurch gekennzeichnet, dass man in Stufe (1) als Carbonsäure eine solche einsetzt, worin R α-verzweigtes Alkyl ist.

3. Verfahren nach Anspruch 2, dadurch gekennzeichnet, dass man als Carbonsäure eine solche einsetzt, worin R α-verzweigtes C₃-C₈-Alkyl ist.

4. Verfahren nach Anspruch 3, dadurch gekennzeichnet, dass man als Carbonsäure eine solche einsetzt, worin R 1-Aethylpropyl, 1-Aethylpentyl oder t-Butyl ist.

5. Verfahren nach Anspruch 1, dadurch gekennzeichnet, dass in Stufe (1) ein saurer oder basischer Katalysator eingesetzt wird.

6. Verfahren nach Anspruch 5, dadurch gekennzeichnet, dass als saurer Katalysator eine Lewis-Säure eingesetzt wird.

7. Verfahren nach Anspruch 6, dadurch gekennzeichnet, dass als saurer Katalysator Eisen-III-chlorid eingesetzt wird.

8. Verfahren nach Anspruch 5, dadurch gekennzeichnet, dass als basischer Katalysator Pyridin, ein mit Alkyl- oder Dialkylaminogruppen substituiertes Pyridin, ein Trialkylamin oder ein polymergebundenes Pyridin oder ein polymergebundenes alkyliertes Aminopyridin eingesetzt wird.

9. Verfahren nach Anspruch 8, dadurch gekennzeichnet, dass als basischer Katalysator Pyridin eingesetzt wird.

10. Verfahren nach Anspruch 1, dadurch gekennzeichnet, dass in Stufe (2) ein Aether eingesetzt wird, worin R₁ Wasserstoff und R₂ C₃-C₅-Alkyl ist.

11. Verfahren nach Anspruch 10, dadurch gekennzeichnet, dass R₂ Isobutyl ist.

12. Verfahren nach Anspruch 1, dadurch gekennzeichnet, dass in Stufe (2) als Katalysator p-Toluolsulfonsäure oder Methansulfonsäure eingesetzt wird.

13. Verfahren nach Anspruch 1, dadurch gekennzeichnet, dass in Stufe (3) als Base Natrium- oder Kaliumhydroxid eingesetzt wird.

14. Verfahren nach Anspruch 1, dadurch gekennzeichnet, dass in Stufe (3) die Hydrolyse und Cyclisierung des Esters in Gegenwart einer Base in einem polaren Lösungsmittel erfolgt.

15. Verfahren nach Anspruch 14, dadurch gekennzeichnet, dass als polares Lösungsmittel Wasser eingesetzt wird.

16. Verfahren nach Anspruch 1, dadurch gekennzeichnet, dass man die in Stufe (3) gebildete Verbindung der Formel VI vor der Acetalspaltung in Stufe (4) durch azeotropes Abdestillieren oder durch Wasserdampfdestillation anreichert.

17. Verfahren nach Anspruch 1, dadurch gekennzeichnet, dass in Stufe (4) als Säure eine Mineralsäure in einem protischen Lösungsmittel eingesetzt wird.

18. Verfahren nach Anspruch 17, dadurch gekennzeichnet, dass in Stufe (4) als Säure Schwefelsäure oder Chlorwasserstoff eingesetzt wird.

19. Verfahren nach Anspruch 1, dadurch gekennzeichnet, dass nach der Acetalspaltung in Stufe (4) die Reaktionsmischung mit einer Base neutralisiert wird.

20. Verfahren nach Anspruch 19, dadurch gekennzeichnet, dass mit einem Alkali- oder Erdalkalihydroxid neutralisiert wird.

21. Verfahren nach Anspruch 1, dadurch gekennzeichnet, dass in Stufe (5) eine Rektifizierung erfolgt.

22. Verfahren nach Anspruch 21, dadurch gekennzeichnet, dass man vor der Rektifizierung eine Extraktion ausführt.

23. Verfahren nach Anspruch 1, dadurch gekennzeichnet, dass man die Stufen (1) bis (5) kontinuierlich ausführt.

24. Verfahren nach Anspruch 1, dadurch gekennzeichnet, dass man eine Verbindung der Formel II einsetzt, worin R₉ und R₁₀ unabhängig voneinander Wasserstoff oder Methyl sind.

25. Verfahren nach Anspruch 24, dadurch gekennzeichnet, dass man eine Verbindung der Formel II einsetzt, worin R₉ und R₁₀ Wasserstoff sind.

## Claims

1. A process for the preparation of 3-hydroxyoxetanes of formula I 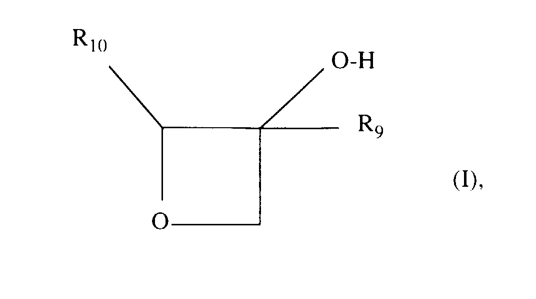 wherein R₉ and R₁₀ are each independently of the other hydrogen or C₁-C₄alkyl, by
(1) reaction of a carboxylic acid R-CO₂H, wherein R is branched alkyl with an epichlorohydrin of formula II 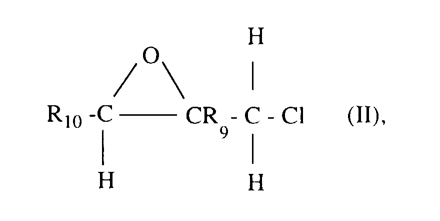 wherein R₉ and R₁₀ are as defined hereinbefore, to form an ester of formula III 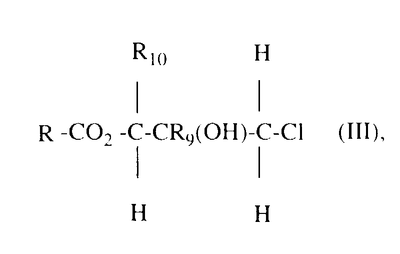 wherein R, R₉ and R₁₀ are as defined hereinbefore,
(2) reaction of that ester with an ether of formula IV
CHR₁=CH-O-R₂ (IV),
wherein R₁ is hydrogen or methyl, R₂ is C₁-C₆alkyl, or R₁ and R₂ together form a radical of formula -(CH₂)₃-, in the presence of a catalyst, to form an ester of formula V 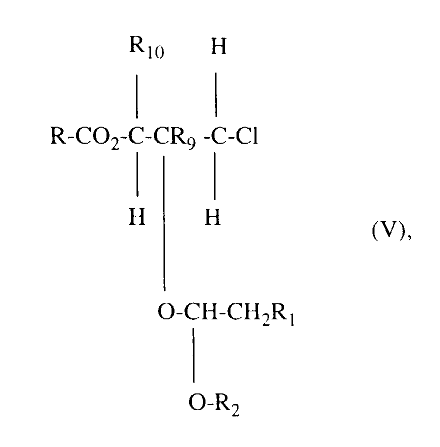
(3) hydrolysis and cyclisation of that ester in the presence of a base to form a compound of formula VI 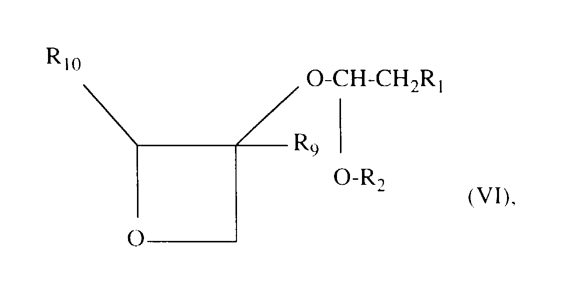 wherein R₁, R₂, R₉ and R₁₀ are as defined hereinbefore,
(4) acetal cleavage in the presence of an acid to form the corresponding 3-hydroxyoxetane and
(5) isolation of that 3-hydroxyoxetane.

2. A process according to claim 1, wherein there is used as carboxylic acid in Step (1) one in which R is α-branched alkyl.

3. A process according to claim 2, wherein there is used as carboxylic acid one in which R is α-branched C₃-C₈alkyl.

4. A process according to claim 3, wherein there is used as carboxylic acid one in which R is 1-ethylpropyl, 1-ethylpentyl or t-butyl.

5. A process according to claim 1, wherein in Step (1) an acidic or basic catalyst is used.

6. A process according to claim 5, wherein there is used as acidic catalyst a Lewis acid.

7. A process according to claim 6, wherein there is used as acidic catalyst iron(III) chloride.

8. A process according to claim 5, wherein there is used as basic catalyst pyridine, a pyridine substituted by alkyl- or dialkyl-amino groups, a trialkylamine or a polymer-bonded pyridine or a polymer-bonded alkylated aminopyridine.

9. A process according to claim 8, wherein pyridine is used as basic catalyst.

10. A process according to claim 1, wherein there is used in Step (2) an ether in which R₁ is hydrogen and R₂ is C₃-C₅alkyl.

11. A process according to claim 10, wherein R₂ is isobutyl.

12. A process according to claim 1, wherein there is used as catalyst in Step (2) p-toluenesulfonic acid or methanesulfonic acid.

13. A process according to claim 1, wherein there is used as base in Step (3) sodium or potassium hydroxide.

14. A process according to claim 1, wherein in Step (3) the hydrolysis and cyclisation of the ester are carried out in the presence of a base in a polar solvent.

15. A process according to claim 14, wherein water is used as polar solvent.

16. A process according to claim 1, wherein the compound of formula VI formed in Step (3) is, before acetal cleavage in Step (4), concentrated by azeotropic distillation or by steam distillation.

17. A process according to claim 1, wherein there is used as acid in Step (4) a mineral acid in a protic solvent.

18. A process according to claim 17, wherein there is used as acid in Step (4) sulfuric or hydrochloric acid.

19. A process according to claim 1, wherein after the acetal cleavage in Step (4) the reaction mixture is neutralised with a base.

20. A process according to claim 19, wherein neutralisation is effected with an alkali metal or alkaline earth metal hydroxide.

21. A process according to claim 1, wherein a rectification is carried out in Step (5).

22. A process according to claim 21, wherein an extraction is carried out before the rectification.

23. A process according to claim 1, wherein Steps (1) to (5) are carried out continuously.

24. A process according to claim 1, wherein a compound of formula II is used in which R₉ and R₁₀ are each independently of the other hydrogen or methyl.

25. A process according to claim 24, wherein a compound of formula II is used in which R₉ and R₁₀ are hydrogen.

## Revendications

1. Un procédé de préparation de 3-hydroxyoxétanes de formule I 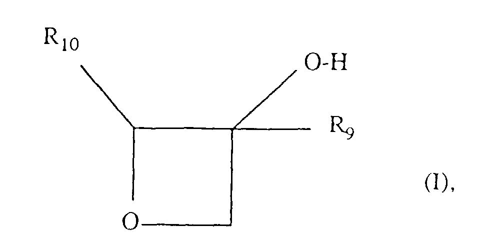 où R₉ et R₁₀ signifient chacun indépendamment l'un de l'autre, l'hydrogène ou un groupe C₁-C₄-alkyle, par
(1) réaction d'un acide carboxylique R-CO₂H, où R signifie un groupe alkyle ramifié, avec une épichlorhydrine de formule Il 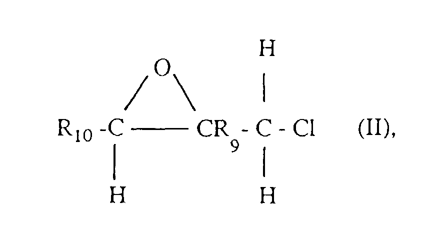 où R₉ et R₁₀ ont les significations données, pour former un ester de formule III 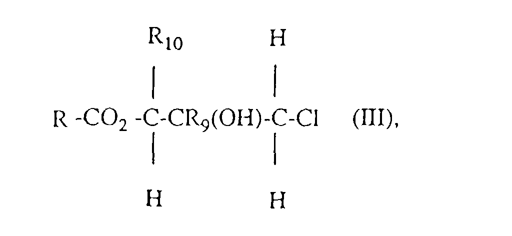 où R, R₉ et R₁₀ ont les significations données,
(2) réaction de cet ester avec un éther de formule IV
CHR₁=CH-O-R₂ (IV),
où R₁ signifie l'hydrogène ou un groupe méthyle, R₂ signifie un groupe C₁-C₆-alkyle, ou R₁ et R₂ forment ensemble un reste de formule -(CH₂)₃-, en présence d'un catalyseur, pour former un ester de formule V 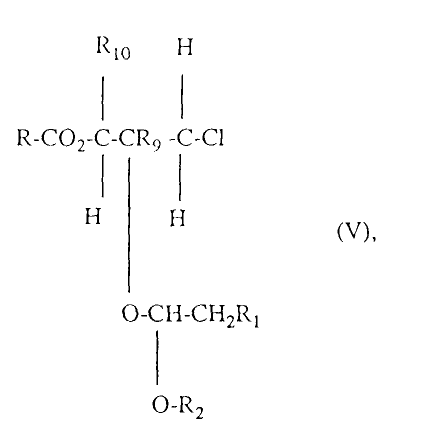
(3) hydrolyse et cyclisation de cet ester en présence d'une base, pour former un composé de formule VI 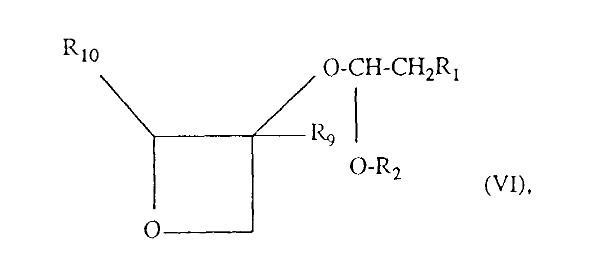 où R₁, R₂, R₉ et R₁₀ ont les significations données,
(4) scission d'acétal en présence d'un acide, pour former le 3-hydroxyoxétane correspondant et,
(5) isolement de ce 3-hydroxyoxétane.

2. Un procédé selon la revendication 1, caractérisé en ce qu'à l'étape (1), on utilise comme acide carboxylique celui dans lequel R signifie un groupe alkyle α-ramifié.

3. Un procédé selon la revendication 2, caractérisé en ce qu'on utilise comme acide carboxylique, celui dans lequel R signifie un groupe C₃-C₈-alkyle α-ramifié.

4. Un procédé selon la revendication 3, caractérisé en ce qu'on utilise comme acide carboxylique, celui dans lequel R signifie un groupe 1-éthylpropyle, 1-éthylpentyle ou tert.-butyle.

5. Un procédé selon la revendication 1, caractérisé en ce qu'à l'étape (1), on utilise un catalyseur acide ou basique.

6. Un procédé selon la revendication 5, caractérisé en ce qu'on utilise comme catalyseur acide, un acide de Lewis.

7. Un procédé selon la revendication 6, caractérisé en ce qu'on utilise comme catalyseur acide, le chlorure de fer(lll).

8. Un procédé selon la revendication 5, caractérisé en ce qu'on utilise comme catalyseur basique, la pyridine, une pyridine substituée par un groupe alkyl- ou dialkylamino, une trialkylamine ou une pyridine liée à un polymère ou une amino-pyridine alkylée liée à un polymère.

9. Un procédé selon la revendication 8, caractérisé en ce qu'on utilise la pyridine comme catalyseur basique.

10. Un procédé selon la revendication 1, caractérisé en ce qu'à l'étape (2), on utilise un éther, dans lequel R₁ signifie l'hydrogène et R₂ signifie un groupe C₃-C₅-alkyle.

11. Un procédé selon la revendication 10, caractérisé en ce que R₂ signifie un groupe isobutyle.

12. Un procédé selon la revendication 1, caractérisé en ce qu'à l'étape (2), on utilise comme catalyseur, l'acide p-toluènesulfonique ou l'acide méthane-sulfonique.

13. Un procédé selon la revendication 1, caractérisé en ce qu'à l'étape (3), on utilise comme base, de l'hydroxyde de sodium ou de potassium.

14. Un procédé selon la revendication 1, caractérisé en ce qu'à l'étape (3), on effectue l'hydrolyse et la cyclisation de l'ester en présence d'une base dans un solvant polaire.

15. Un procédé selon la revendication 14, caractérisé en ce qu'on utilise l'eau comme solvant polaire.

16. Un procédé selon la revendication 1, caractérisé en ce qu'on concentre le composé de formule VI formé à l'étape (3), avant la scission de l'acétal à l'étape (4), par distillation azéotropique ou par distillation à la vapeur.

17. Un procédé selon la revendication 1, caractérisé en ce qu'à l'étape (4), on utilise comme acide, un acide minéral dans un solvant protique.

18. Un procédé selon la revendication 17, caractérisé en ce qu'à l'étape (4), on utilise comme acide, l'acide sulfurique ou l'acide chlorhydrique.

19. Un procédé selon la revendication 1, caractérisé en ce que, après la scission de l'acétal à l'étape (4), on neutralise le mélange réactionnel avec une base.

20. Un procédé selon la revendication 19, caractérisé en ce qu'on effectue la neutralisation avec un hydroxyde de métal alcalin ou de métal alcalino-terreux.

21. Un procédé selon la revendication 1, caractérisé en ce qu'à l'étape (5), on effectue une rectification.

22. Un procédé selon la revendication 21, caractérisé en ce qu'on effectue une extraction avant la rectification.

23. Un procédé selon la revendication 1, caractérisé en ce qu'on effectue les étapes (1) à (5) en continu.

24. Un procédé selon la revendication 1, caractérisé en ce qu'on utilise un composé de formule II, dans lequel R₉ et R₁₀ signifient chacun indépendamment l'un de l'autre, l'hydrogène ou un groupe méthyle.

25. Un procédé selon la revendication 24, caractérisé en ce qu'on utilise un composé de formule II, dans lequel R₉ et R₁₀ signifient l'hydrogène.
